# EUROPEAN PATENT SPECIFICATION

(11) **EP 3 144 370 B1**
(45) Date of publication and mention of the grant of the patent: **14.11.2018**
(21) Application number: 16189362.3
(22) Date of filing: 16.09.2016
(51) Int. Cl.: C10L 1/222, C10L 1/2387, C10L 10/08, C10L 10/14, C10L 1/22

(54) **USE OF A FUEL COMPOSITION**
VERWENDUNG EINER KRAFTSTOFFZUSAMMENSETZUNG
UTILISATION D'UNE COMPOSITION DE CARBURANT

(30) Priority: 16.09.2015 US 201514855674
(43) Date of publication of application: 22.03.2017
(73) Proprietor: Afton Chemical Corporation, Richmond, VA 23219 (US)
(72) Inventor: FANG, Xinggao, Midlothian, VA Virginia 23114 (US); CULLEY, Scott A., Midlothian, VA Virginia 23113 (US); SCHWAB, Scott D., Richmond, VA Virginia 23233 (US)
(74) Representative: Schwabe - Sandmair - Marx

(56) References cited:
- EP-A1- 0 869 163
- EP-A1- 0 887 401
- WO-A2-01/42188
- US-A- 4 231 883
- US-A- 5 678 631

## Description

### TECHNICAL FIELD:

The disclosure is directed to use of a gasoline fuel composition that improves fuel economy. In particular, the disclosure relates to polyhydroxyalkyl ether amine additives that reduce friction or wear of engine parts and improve fuel economy of an engine while remaining stable in a fuel additive package.

### BACKGROUND AND SUMMARY:

Fuel compositions for vehicles are continually being improved to enhance various properties of the fuels in order to accommodate their use in newer, more advanced engines including direct injection gasoline engines. Accordingly, fuel compositions typically include additives that are directed to certain properties that require improvement. For example, friction modifiers, such as fatty acid amides, are added to fuel to reduce friction and wear in the fuel delivery systems of an engine. However, certain fatty amides may be unstable in additive packages for fuels at low storage temperatures and the performance of such fatty acid amides is often less than desirable. Fuel additives may be passed into the oil sump during engine operation, so that a fuel additive that is also beneficial to the engine lubricant is desirable. While such additives may be beneficially added to the lubricant rather than the fuel, such additive are not effective for improving wear in fuel delivery systems. Also, such additives, when added to the fuel, rather than the lubricant, may reduce friction and wear in the piston ring zone of the engine and thus improve fuel economy. Accordingly, it is beneficial to include additives in fuels to provide both improved fuel delivery system wear protection as well as improved fuel economy.

Partial esters of fatty acid and polyhydroxy alcohols such as glycerol monooleate (GMO) are known as friction modifiers for lubricant compositions. Likewise diethanolamine fatty amides are also well known friction modifiers. While GMO and fatty amide friction modifiers may improve fuel economy when added to a lubricant, GMO and certain diethanolamine fatty amides may be unstable in additive packages for fuels or may cause an increase in intake valve deposits in gasoline engines. Furthermore, fuel economy improvement may be less than desirable when using GMO or certain fatty amides in fuel compositions. Accordingly, GMO and fatty amide friction modifiers cannot be beneficially added to a fuel composition to improve the wear protection of the fuel delivery system without harmful and undesirable side effects.

Fatty amine ethoxylates are also known as fuel additives that may reduce fuel consumption. However, such fatty amine ethoxylates are typically derived from natural sources and thus may vary by region and over time. In addition, some fatty amine ethoxylates have high freezing points or are solids at room temperature and may require heating or the use of a solvent for storage and handling. Lastly, fatty amine ethoxylates typically have poor low temperature compatibility with fuel compositions.

Certain etheramine polyalkoxylates were believed to be useful as anticorrosion additives in gasoline fuels. However, such compounds perform poorly with regard to corrosion in a NACE TM0172-2001 corrosion test and may dramatically increase the amount of intake valve deposits in an engine.

Many other friction modifiers have been tried, however there remains a need for a friction modifier that enables a fuel additive packages containing the friction modifier to achieve one or more of remaining fluid at low temperatures, being resistant to hydrolysis, that may be readily formulated into a fuel additive packages, that offer good fuel economy benefits, and that provide wear protection to fuel delivery systems, among other characteristics. Accordingly, there continues to be a need for a fuel additive that is cost effective to manufacture and improves at least one and preferably multiple characteristics of a fuel.

In accordance with the disclosure, exemplary embodiments
are directed to a use of a fuel composition for improving fuel economy of an internal combustion engine, the fuel composition comprising gasoline and from 10 to 750 ppm by weight based on the total weight of the fuel composition of an additive of the formula: 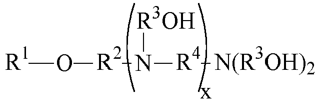 wherein R¹ comprises a saturated hydrocarbyl group having from 6 to 30 carbon atoms, R² is a linear alkylene group, or an alkoxyalkylene group, or a polyalkoxyalkylene group wherein the alkylene contains from 2 to 25 carbon atoms, R³ is an alkylene group containing from 2 to 5 carbon atoms, R⁴ is a linear alkylene group containing 2 to 3 carbon atoms, and x is an integer selected from 0 and 1.

An advantage of the methods described herein is that the additive for the fuel composition may not only improve the friction or wear properties of the fuel, but the additive may also be effective to improve fuel economy without detrimentally affecting the low temperature stability of a fuel additive package containing the additive component.

In one embodiment, the additive is derived from a hydrocarbyl substituted ether amine that is reacted with an epoxide. In another embodiment, the hydrocarbyl group of the hydrocarbyl substituted ether amine contains from 6 to 30 carbon atoms.

In one embodiment, the hydrocarbyl ether amine is a compound of the formula 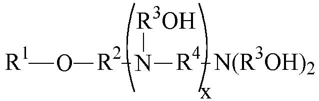 wherein R¹ comprises a saturated hydrocarbyl group having from 6 to 30 carbon atoms, R² is a linear alkylene group, a linear polyalkylene group, an alkoxyalkylene group, or a polyalkoxyalkylene group containing from 2 to 25 carbon atoms, R³ is an alkylene group containing from 2 to 5 carbon atoms, R⁴ is a linear alkylene group containing 2 to 3 carbon atoms, and x is an integer selected from 0 and 1.

In a further embodiment, the fuel composition contains from 10 to 750 ppm by weight, such as from 40 to 500 ppm by weight, or from 50 to 250 ppm by weight of the polyhydroxyalkyl ether amine based on a total weight of the fuel composition.

Additional embodiments and advantages of the disclosure will be set forth in part in the detailed description which follows, and/or can be learned by practice of the disclosure. It is to be understood that both the foregoing general description and the following detailed description are exemplary and explanatory only and are not restrictive of the disclosure, as claimed. The invention is set out in the appended set of claims.

EP 0 869 163 A1 relates to methods for reducing friction in the operation of an internal combustion engine comprising delivering to the internal combustion engine a fuel comprising gasoline and a friction-reducing additive based on a N,N-bis(hydroxyalkyl)-alkylamine.

U.S. Patent no. 4,231,883 A relates to reducing the friction of sliding metal surfaces in an internal combustion engine by using a lubricating oil in the engine crankcase which contains an alkoxylated aliphatic hydrocarbyl amine.

### DETAILED DESCRIPTION OF EXEMPLARY EMBODIMENTS

The fuel additive component of the present application may be used in a minor amount in a major amount of fuel and may be added to the fuel directly or added as a component of an additive concentrate to the fuel. A suitable fuel additive component for improving the operation of internal combustion engines may be made by reacting an ether amine or ether diamine with an epoxide.

As used herein, the term "hydrocarbyl group" or "hydrocarbyl" is used in its ordinary sense, which is well-known to those skilled in the art. Specifically, it refers to a group having a carbon atom directly attached to the remainder of a molecule and having a predominantly hydrocarbon character. Examples of hydrocarbyl groups include:
(1) hydrocarbon substituents, that is, aliphatic (e.g., alkyl or alkenyl), alicyclic (e.g., cycloalkyl, cycloalkenyl) substituents, and aromatic-, aliphatic-, and alicyclic-substituted aromatic substituents, as well as cyclic substituents wherein the ring is completed through another portion of the molecule (e.g., two substituents together form an alicyclic radical);
(2) substituted hydrocarbon substituents, that is, substituents containing non-hydrocarbon groups which, in the context of the description herein, do not alter the predominantly hydrocarbon substituent (e.g., halo (especially chloro and fluoro), hydroxy, alkoxy, mercapto, alkylmercapto, nitro, nitroso, amino, alkylamino, and sulfoxy);
(3) hetero-substituents, that is, substituents which, while having a predominantly hydrocarbon character, in the context of this description, contain other than carbon in a ring or chain otherwise composed of carbon atoms. Hetero-atoms include sulfur, oxygen, nitrogen, and encompass substituents such as pyridyl, furyl, thienyl, and imidazolyl. In general, no more than two, or as a further example, no more than one, non-hydrocarbon substituent will be present for every ten carbon atoms in the hydrocarbyl group; in some embodiments, there will be no non-hydrocarbon substituent in the hydrocarbyl group.

As used herein, the term "major amount" is understood to mean an amount greater than or equal to 50 wt. %, for example from 80 to 98 wt .% relative to the total weight of the composition. Moreover, as used herein, the term "minor amount" is understood to mean an amount less than 50 wt. % relative to the total weight of the composition.

### Ether-Amine Compound

According to the disclosure, any suitable ether-amine or ether-diamine may be used to prepare a compound of the formula: 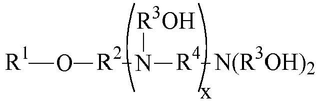 wherein R¹ comprises a saturated hydrocarbyl group having from 6 to 30 carbon atoms, R² is a linear alkylene group, an alkoxyalkylene group, or a polyalkoxyalkylene group containing from 2 to 25 carbon atoms, R³ is an alkylene group containing from 2 to 5 carbon atoms, R⁴ is a linear alkylene group containing 2 to 3 carbon atoms, and x is an integer selected from 0 and 1. In one embodiment, the amine may be a compound of the formula:

R¹-O-R²-(NH-R⁴)ₓ-NH₂

wherein R¹ comprises a saturated hydrocarbyl group having from 6 to 30 carbon atoms, R² is an alkylene group, polyalkylene group, alkoxyalkylene group, or polyalkoxyalkylene group containing from 2 to 25 carbon atoms, R⁴ is a linear alkylene group containing 2 to 3 carbon atoms, and x is an integer selected from 0 and 1.

Representative etheramines may include, but are not limited to, isohexyloxyethylamine, isohexyloxypropylamine, 2-ethylhexyloxyethylamine, 2-ethylhexyloxypropylamine, a mixture of octyloxyethylamine and decyloxyethylamine, a mixture of octyloxypropylamine and decyloxypropylamine, isodecyloxyethylamine, isodecyloxypropylamine, isododecyloxyethylamine, isododecyloxypropylamine, isotridecyloxyethylamine, isotridecyloxypolyproxypropylamine, isotridecyloxypoly-C₂-C₄-oxypropylamine, isotridecyloxypropylamine, C₁₂-C₁₅-alkyloxyethylamine, C₁₂-C₁₅-alkyloxypropylamine, C₁₆-C₁₈-alkyloxyethylamine, C₁₆-C₁₈-alkyloxypropylamine, isodecyloxyethyl-1,3-diaminopropane, isodecyloxypropyl-1,3-diaminopropane, isododecyloxyethyl-1,3-diaminopropane, isododecyloxypropyl-1,3-diaminopropane, isotridecyloxyethyl-1,3-diaminopropane, isotridecyloxypropyl-1,3-diaminopropane, and the like.

### Epoxide Compound

A suitable epoxide compound may be selected from the group consisting of:
1,3-Butadiene diepoxide
Cyclohexene oxide
Cyclopentene oxide
Dicyclopentadiene dioxide
1,2,5,6-Diepoxycyclooctane
1,2,7,8-Diepoxyoctane
1,2-Epoxybutane
cis-2,3-Epoxybutane
3,4-Epoxy-1-butene
3,4-Epoxycyclohexylmethyl 3,4-epoxycyclohexanecarboxylate
1,2-Epoxydodecane
1,2-Epoxyhexadecane
1,2-Epoxyhexane
1,2-Epoxy-5-hexene
1,2-Epoxy-2-methylpropane
exo-2,3-Epoxynorbornane
1,2-Epoxyoctane
1,2-Epoxypentane
1,2-Epoxy-3-phenoxypropane
(2,3-Epoxypropyl)benzene
N-(2,3-Epoxypropyl)phthalimide
1,2-Epoxytetradecane
exo-3,6-Epoxy-1,2,3,6-tetrahydrophthalic anhydride
3,4-Epoxytetrahydrothiophene-1,1-dioxide
Isophorone oxide
Methyl-1,2-cyclopentene oxide
2-Methyl-2-vinyloxirane
α-Pinene oxide
Ethylene oxide
(±)-propylene oxide
Polyisobutene oxide
cis-Stilbene oxide
Styrene oxide
Glycidol
Glycidol ethers
Tetracyanoethylene oxide
Tris(2,3-epoxypropyl) isocyanurate and combinations of two or more of the foregoing. A particularly suitable epoxide may be selected from ethylene oxide, propylene oxide, and glycidol.

The polyhydroxy ether amines may be made in one stage or two stages. The reaction may be carried out by contacting and mixing an alcohol or ether alcohol with an epoxide to form an alkoxylated alcohol, aminating the alkoxylated alcohol with ammonia in the presence of a catalyst to form an alkoxylated alkylamine, and subsequently reacting the alkoxylated alkylamine with an epoxide to form the alkoxylated ether amines. The mole ratio of alcohol to epoxide may range from 1 to 8, such as a mole ratio of alcohol to epoxide ranging from 2 to 4. The mole ratio of ammonia or amine to alkoxylated alcohol may range from 1 to 10. The mole ratio of epoxide to alkoxylated alkylamine may range from 1 to 5. In one embodiment, only one epoxy group is added to each NH group. The reactions may be conducted at temperatures ranging from 0° C to 160° C. In another embodiment, the ether amine could be made by reacting a hydrocarbyl ether with acrylonitrile followed by reduction of the resulting intermediate.

One or more additional optional compounds may be present in the fuel compositions of the disclosed embodiments. For example, the fuels may contain conventional quantities of octane improvers, corrosion inhibitors, cold flow improvers (CFPP additive), pour point depressants, solvents, demulsifiers, lubricity additives, additional friction modifiers, amine stabilizers, combustion improvers, dispersants, detergents, antioxidants, heat stabilizers, conductivity improvers, metal deactivators, carrier fluid, marker dyes, organic nitrate ignition accelerators, cyclomatic manganese tricarbonyl compounds, and the like. In some aspects, the compositions described herein may contain 10 weight percent or less, or in other aspects, 5 weight percent or less, based on the total weight of the additive concentrate, of one or more of the above additives. Similarly, the fuels may contain suitable amounts of conventional fuel blending components such as methanol, ethanol, dialkyl ethers, 2-ethylhexanol, and the like.

In one embodiment, a fuel additive package may contain the above described polyhydroxyalkyl ether amine additive in combination with a carrier fluid and other ingredients selected from fatty amine ethoxylates; one or more detergents selected from Mannich bases, polyalkylamines, polyalkylpolyamines, polyalkenyl succinimides, and quaternary ammonium salt detergents. Quaternary ammonium salt detergents may be selected from compounds of the formula 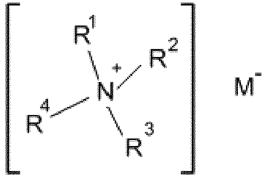 wherein each of R¹, R², R³, and R⁴ is selected from a hydrocarbyl group containing from 1 to 50 carbon atoms, wherein at least one and not more than three of R¹, R², R³, and R⁴ is a hydrocarbyl group containing from 1 to 4 carbon atoms and at least one of R¹, R², R³, and R⁴ is a hydrocarbyl group containing from 8 to 50 carbon atoms, M⁻ is selected from ions of the group consisting of carboxylates, nitrates, nitrides, nitrites, hyponitrites, phenates, carbamates, carbonates, and mixtures thereof, wherein the carboxylate is not an oxalate or formate; alkoxylated quaternary ammonium salts derived from epoxides, tertiary amines, and optional protonating agents; reaction products of amido amines or acylated amines containing at least one tertiary amino group and epoxides; reaction products of hydrocarbyl substituted anhydrides, tertiary amines and hydroxyl-containing epoxides; esterified quaternary ammonium salts derived from tertiary amines, epoxides, proton donors and anhydrides; reaction products of hydrocarbyl substituted compounds containing at least one tertiary amino group selected from C₁₀-C₃₀-alkyl or alkenyl-substituted amidopropyldimethylamines and C₁₂-C₂₀₀-alkyl or alkenyl-substituted succinic-carbonyldimethylamines and halogen substituted C₂-C₈ carboxylic acids, esters, amides, or salts thereof; and mixtures two or more of the foregoing detergents.

Suitable carrier fluids may be selected from any suitable carrier fluid that is compatible with the fuel or gasoline and is capable of dissolving or dispersing the components of the additive package. Typically the carrier fluid is a hydrocarbyl polyether or a hydrocarbon fluid, for example a petroleum or synthetic lubricating oil basestock including mineral oil, synthetic oils such as polyesters or polyethers or other polyols, or hydrocracked or hydroisomerised basestock. Alternatively the carrier fluid may be a distillate boiling at a temperature in a range of boiling temperatures of the fuel or gasoline of from about 30 to 200°C or from about 35 to 175°C. The amount of carrier fluid contained in the additive package may range from 10 to 80 wt %, preferably from 20 to 75 wt %, and more preferably from 30 to 60 wt % based on a total weight of the additive package. Such additive packages containing the polyhydroxyalkyl ether amine additive, detergent and carrier fluid were found to remain as clear fluids even at temperatures as low as -20 to -30° C.

The additives of the present application, including the polyhydroxylalkyl ether amines described above, and optional additives used in formulating the fuels of this invention may be blended into the base fuel individually or in various sub-combinations. In some embodiments, the additive components of the present application may be blended into the fuel concurrently using an additive concentrate, as this takes advantage of the mutual compatibility and convenience afforded by the combination of ingredients when in the form of an additive concentrate. Also, use of a concentrate may reduce blending time and lessen the possibility of blending errors.

The fuels of the present application may be applicable to the operation of gasoline engines. The engine include both stationary engines (e.g., engines used in electrical power generation installations, in pumping stations, etc.) and ambulatory engines (e.g., engines used as prime movers in automobiles, trucks, road-grading equipment, military vehicles, etc.).

### EXAMPLES

The following examples are illustrative of exemplary embodiments of the disclosure. In these examples as well as elsewhere in this application, all parts and percentages are by weight unless otherwise indicated. It is intended that these examples are being presented for the purpose of illustration only and are not intended to limit the scope of the invention disclosed herein.

In the following example, a friction test was conducted on a GF-5 lubricating oil that was devoid of friction modifiers using a high frequency reciprocating rig (HFRR) under a 4N load with a stroke distance of 1 millimeter at 20 Hz at 70° C, 100° C and 130° C according to ASTM D6079. The base lubricating oil contained a GF-5 dispersant/inhibitor (DI) package that contained no friction modifiers.

Inventive additives were made as follows:

### Inventive Additive 1

An alkyl amine ethoxylate, C₁₃O(PO)₃N(EO)₂, made by reaction of C₁₃OH with propylene oxide followed by reductive amination and was reacted with 2 equivalents of ethylene oxide to yield Inventive Additive 1.

### Inventive Additive 2

Inventive Additive 2 was made using the process of Inventive Example 1 except that C₁₃OH was replaced by a mixture of C₁₆ and C₁₈ alcohols.

### Inventive Example 3

Inventive Example 3 was bis(2-hydroxyethyl) isotridecyloxylpropylamine.

### Inventive Example 4

Inventive Example 4 was a reaction product of a mixture of dodecylcycloxypropyl-1,3-diamino propane and tetradecloxypropyl-1,3-diaminopropane with three equivalents of ethylene oxide.

The treat rate of the additive and the results are given in the following table.

**Table 1 - Oil HFRR data**

| **Ex. No.** | **Additive** | **Treat rate (wt.%)** | **Coefficients of friction at 130° C** |
|---|---|---|---|
| **1** | Base lubricant plus DI package | 0 | 0.159 |
| **2** | No. 1 plus additive of Inventive Ex. 1 | 0.125 | 0.123 |
| **3** | No. 1 plus additive of Inventive Ex. 2 | 0.125 | 0.116 |
| **4** | No. 1 plus additive of Inventive Ex. 3 | 0.125 | 0.139 |

Some of the additive in fuel is transferred into the lubricant within the piston area between the liner and the ring and accumulates in the lubricant in the oil sump over time. Thus, the performance of the inventive examples in reducing the coefficient of friction as shown in Table 1 is indicative of the beneficial effect of the present invention on friction and wear in the piston ring zone as well as reducing friction in the other engine components. As shown by the foregoing examples, the inventive examples (Runs 2-4) exhibited reduced friction in a lubricant composition compared the base lubricant devoid of friction modifiers

### Modified Sequence VIE Dynamometer Testing

The following results were from top-treat additions of friction modifiers to a Sequence VIE motor oil (Table 2) and fuel (Table 3) while the engine was operating at high temperature and load. The modified Sequence VIE testing was carried out using a General Motors 3.6L (LY7) V6, 4-cycle engine equipped with dual overhead camshafts and having four valves per cylinder and also equipped with a dual stage Plenum induction manifold with a 94 x 85.6 mm bore & stroke with a 10.2:1 compression ratio. The test fuel was the Sequence VI E reference fuel and the motor oil was a formulated SAE 0W-20 passenger car engine oil containing all of the standard engine oil components, but containing no friction modifiers. To make the top-treated lubricant, the friction modifier to be tested was solubilized in the Sequence VIE motor oil. The concentration of friction modifier in the top-treat was sufficient to provide a concentration of 0.125 wt.% of friction modifier in the crankcase lubricant. The engine was operated with the baseline engine oil at 1500 rpm, a torque of 150 N-m, an oil temperature of 115° C and a coolant temperature of 109° C until the temperatures stabilized. The brake specific fuel consumption (BSFC) was measured for approximately one hour after stabilization. The top-treated lubricant containing the friction modifier was then added to the crankcase. Upon the addition of the top-treated lubricant, the BSFC decreased over the course of about five minutes. After the BSFC stabilized, the fuel consumption was measured for approximately one hour. The fuel economy improvement was calculated from the average BSFC before and after the addition of the friction modifier top-treat.

**Table 2 - Fuel Economy Increase**

| **Run No.** | **Friction Modifier in engine oil** | **% Fuel Economy Increase** |
|---|---|---|
| **1** | Base oil, plus no top treat additive | 0 |
| **2** | Base oil, plus glycerol monooleate | 0.42 |
| **3** | Base oil, plus - diethanolamine fatty amide derived from fatty acid and diethanol amine | 0.24 |
| **4** | Base oil plus Inventive Example 1 | 1.19 |
| **5** | Base oil plus Inventive Example 2 | 1.08 |
| **6** | Base oil plus Inventive Example 3 | 0.93 |
| **7** | Base oil plus Inventive Example 4 | 1.02 |

The results in Table 3 were obtained from fuels dosed with friction modifiers. Each fuel was dosed with 480 ppm of the friction modifier tested and the fuel economy was determined in the same manner as with the top-treated lubricant runs.

**Table 3 - Fuel Economy Increase**

| **Run No.** | **Friction Modifier dosed into the fuel at a concentration of 480ppm** | **% Fuel Economy Increase** |
|---|---|---|
| **8** | Fuel plus - diethanolamine fatty amide derived from fatty acid and diethanol amine | <0.2 |
| **9** | Fuel plus Inventive Example 1 | 0.4 |

As shown in the foregoing tables, all of the inventive examples provided a significant fuel economy increase in an engine oil composition compared to the base oil composition that was devoid of the inventive friction modifiers. The fuel dosed with the friction modifier of Inventive Example 1 provided a significantly better fuel economy increase than the fuel of Run 2 containing a diethanolamine fatty amide.

An important characteristic of the fuel additives of the disclosure is the low temperature stability of a fuel additive package containing the above described polyhydroxy ether amine. An advantage of providing the additive in a fuel additive package rather than in a lubricant composition is that the additive is continually renewed over time as fuel is combusted in the engine. By contrast, as the lubricant ages, additives provided by the lubricant are typically depleted over time. Accordingly, in order to provide sufficient additive to a fuel to improve the fuel economy of an engine, the additive package containing the foregoing polyhydroxy ether amine must be stable in the fuel additive package. By "stable" is meant the additive package remains a clear fluid at temperatures as low as -20° C over a period of time.

In the following example, the storage stability of conventional gasoline fuel additive packages containing the compounds of Inventive Examples 1-3 (Ex. Nos. 4-6) were compared to additive packages containing conventional friction modifiers. All of the samples in the following table contained 53.85 wt.% of the conventional gasoline fuel additive package and the amount of additive and solvent shown.

**Table 4 - Storage Stability at -20°C**

| **Ex. No.** | **Additive** | **Treat rate (wt.%)** | **Solvent Wt.%** | **1 day** | **1 week** |
|---|---|---|---|---|---|
| **1** | Cocoamine diethoxylate in aromatic solvent | 30.77 | 15.38 | frozen | frozen |
| **2** | Tallowamine diethoxylate in aromatic solvent | 30.77 | 15.38 | frozen | frozen |
| **3** | diethanolamine fatty amide derived from fatty acid and diethanol amine | 30.77 | 15.38 | Clear and bright | Clear and bright |
| **4** | Inventive Ex. 1 in aromatic solvent | 30.77 | 15.38 | Clear and bright | Clear and bright |
| **5** | Inventive Ex. 2 in aromatic solvent | 30.77 | 15.38 | Clear and bright | Clear and bright |
| **6** | Inventive Ex. 3 in aromatic solvent | 30.77 | 15.38 | Clear and bright | Clear and bright |
| **7** | Mixture of cocoamine diethoxylate and Inventive Ex. 1 in aromatic solvent (1:2 weight ratio) | 10.26/20.5 | 15.38 | Clear and bright | Clear and bright |
| **8** | Mixture of cocoamine diethoxylate and Inventive Ex. 1 in aromatic solvent (1:1 weight ratio) | 15.38/15.38 | 15.38 | Clear and bright | Clear and bright |

As shown in the foregoing table, all of the Inventive Examples 1-3 remained clear and bright after a week at a temperature of -20°C (Ex. Nos. 4-6) compared to conventional friction modifiers Ex. Nos. 1-2. Furthermore, Ex. Nos. 7-8, showed that a combination of inventive friction modifier and conventional friction modifier may be used to improve the low temperature storage stability of a conventional friction modifier in a fuel additive composition.

It is noted that, as used in this description the singular forms "a," "an," and "the," include plural referents unless expressly and unequivocally limited to one referent. Thus, for example, reference to "an antioxidant" includes two or more different antioxidants. As used herein, the term "include" and its grammatical variants are intended to be nonlimiting, such that recitation of items in a list is not to the exclusion of other like items that can be substituted or added to the listed items

Unless indicated to the contrary, the numerical parameters set forth in the description are approximations that may vary depending upon the desired properties sought to be obtained by the present disclosure. Each numerical parameter should at least be construed in light of the number of reported significant digits and by applying ordinary rounding techniques. Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

It is to be understood that each component, compound, substituent, or parameter disclosed in the description is to be interpreted as being disclosed for use alone or in combination with one or more of each and every other component, compound, substituent, or parameter disclosed in the description.

It is also to be understood that each amount/value or range of amounts/values for each component, compound, substituent, or parameter disclosed in the description is to be interpreted as also being disclosed in combination with each amount/value or range of amounts/values disclosed for any other component(s), compounds(s), substituent(s), or parameter(s) disclosed in the description and that any combination of amounts/values or ranges of amounts/values for two or more component(s), compounds(s), substituent(s), or parameters disclosed description are thus also disclosed in combination with each other for the purposes of this description.

It is further understood that each lower limit of each range disclosed in the description is to be interpreted as disclosed in combination with each upper limit of each range disclosed in the description for the same component, compounds, substituent, or parameter. Thus, a disclosure of two ranges is to be interpreted as a disclosure of four ranges derived by combining each lower limit of each range with each upper limit of each range. A disclosure of three ranges is to be interpreted as a disclosure of nine ranges derived by combining each lower limit of each range with each upper limit of each range, etc. Furthermore, specific amounts/values of a component, compound, substituent, or parameter disclosed in the description is to be interpreted as a disclosure of either a lower or an upper limit of a range and thus can be combined with any other lower or upper limit of a range or specific amount/value for the same component, compound, substituent, or parameter disclosed elsewhere in the description to form a range for that component, compound, substituent, or parameter.

## Claims

1. Use of a fuel composition for improving fuel economy of an internal combustion engine, the fuel composition comprising gasoline and from 10 to 750 ppm by weight based on the total weight of the fuel composition of an additive of the formula: 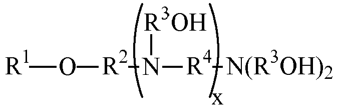 wherein R¹ is a saturated hydrocarbyl group having from 6 to 30 carbon atoms, R² is a linear alkylene group, a alkoxyalkylene group, or a polyalkoxyalkylene group wherein the alkylene contains from 2 to 25 carbon atoms, R³ is an alkylene group containing from 2 to 5 carbon atoms, R⁴ is a linear alkylene group containing 2 to 3 carbon atoms, and x is an integer selected from 0 and 1.

2. The use of claim 1, wherein the fuel composition contains from 40 to 250 ppm by weight of the additive based on a total weight of the fuel composition.

3. The use of any one of claims 1-2, wherein R¹ is a saturated hydrocarbyl group having from 8 to 18 carbon atoms.

4. The use of any one of claims 1-2, wherein R² is -CH₂CH₂CH₂-.

5. The use of any one of claims 1-3, wherein x is 1 and R² and R⁴ are
-CH₂CH₂CH₂-.

6. The use of any one of claims 1-3, wherein R² is a linear alkylene or an alkoxyalkylene group containing from 2 to 10 carbon atoms.

7. The use of any one of claims 1-3, wherein x is 0 and R² is -CH₂CH₂CH₂-.

8. The use of any one of claims 1-7, wherein the fuel composition further comprises a fatty amine diethoxylate, wherein a weight ratio of the additive to the fatty amine diethoxylate ranges from 1:1 to 2:1.

## Patentansprüche

1. Verwendung einer Kraftstoffzusammensetzung zur Verbesserung des Kraftstoffverbrauchs eines Verbrennungsmotors, wobei die Kraftstoffzusammensetzung Benzin und 10 bis 750 ppm, bezogen auf das Gesamtgewicht der Kraftstoffzusammensetzung, eines Additivs folgender Formel umfasst: 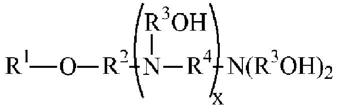 wobei R¹ eine gesättigte Kohlenwasserstoffgruppe mit 6 bis 30 Kohlenstoffatomen ist, R² eine lineare Alkylengruppe, eine Alkoxyalkylengruppe oder eine Polyalkoxyalkylengruppe ist, wobei das Alkylen 2 bis 25 Kohlenstoffatome enthält, R³ eine Alkylengruppe mit 2 bis 5 Kohlenstoffatomen ist, R⁴ eine lineare Alkylengruppe mit 2 bis 3 Kohlenstoffatomen ist und x eine Ganzzahl ist, ausgewählt aus 0 und 1.

2. Verwendung von Anspruch 1, wobei die Kraftstoffzusammensetzung 40 bis 250 ppm, bezogen auf das Gesamtgewicht der Kraftstoffzusammensetzung, enthält.

3. Verwendung eines der Ansprüche 1-2, wobei R¹ eine gesättigte Kohlenwasserstoffgruppe mit 8 bis 18 Kohlenstoffatomen ist.

4. Verwendung eines der Ansprüche 1-2, wobei R²-CH₂CH₂CH₂- ist.

5. Verwendung eines der Ansprüche 1-3, wobei x 1 ist und R² und R⁴ -CH₂CH₂CH₂- sind.

6. Verwendung eines der Ansprüche 1-3, wobei R² eine lineare Alkylen- oder Alkoxyalkylengruppe mit 2 bis 10 Kohlenstoffatomen ist.

7. Verwendung eines der Ansprüche 1-3, wobei x 0 ist und R² -CH₂CH₂CH₂- ist.

8. Verwendung eines der Ansprüche 1-7, wobei die Kraftstoffzusammensetzung ferner ein Fettamindiethoxylat umfasst, wobei das Massenverhältnis des Additivs zu dem Fettamindiethoxylat im Bereich von 1:1 bis 2:1 liegt.

## Revendications

1. Utilisation d'une composition de carburant pour améliorer l'économie de carburant d'un moteur à combustion interne, la composition de carburant comprenant de l'essence et de 10 à 750 ppm en poids sur la base du poids total de la composition de carburant d'un additif de formule : 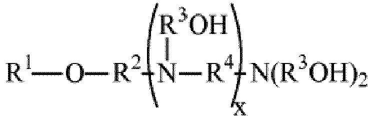 dans laquelle R¹ est un groupe hydrocarbyle saturé ayant de 6 à 30 atomes de carbone, R² est un groupe alkylène linéaire, un groupe alcoxyalkylène, ou un groupe polyalcoxyalkylène dans lequel l'alkylène contient de 2 à 25 atomes de carbone, R³ est un groupe alkylène contenant de 2 à 5 atomes de carbone, R⁴ est un groupe alkylène linéaire contenant de 2 à 3 atomes de carbone et x est un entier choisi parmi 0 et 1.

2. Utilisation selon la revendication 1, où la composition de carburant contient de 40 à 250 ppm en poids de l'additif sur la base d'un poids total de la composition de carburant.

3. Utilisation selon l'une quelconque des revendications 1 à 2, où R¹ est un groupe hydrocarbyle saturé ayant de 8 à 18 atomes de carbone.

4. Utilisation selon l'une quelconque des revendications 1 à 2, où R² est -CH₂CH₂CH₂-.

5. Utilisation selon l'une quelconque des revendications 1 à 3, où x vaut 1 et R² et R⁴ sont
-CH₂CH₂CH₂-.

6. Utilisation selon l'une quelconque des revendications 1 à 3, où R² est un alkylène linéaire ou un groupe alcoxyalkylène contenant de 2 à 10 atomes de carbone.

7. Utilisation selon l'une quelconque des revendications 1 à 3, où x vaut 0 et R² est -CH₂CH₂CH₂-.

8. Utilisation selon l'une quelconque des revendications 1 à 7, où la composition de carburant comprend en outre une amine grasse diéthoxylée, un rapport massique de l'additif à l'amine grasse diéthoxylée allant de 1:1 à 2:1.
